# EUROPEAN PATENT SPECIFICATION

(11) **EP 3 255 711 B1**
(45) Date of publication and mention of the grant of the patent: **31.03.2021**
(21) Application number: 15881034.1
(22) Date of filing: 10.12.2015
(51) Int. Cl.: H01G 11/46, H01G 11/04, H01G 11/86, H01M 4/46, C25D 3/42, H01M 4/134, H01M 4/1395, H01G 11/30, H01M 10/054, H01M 4/04, H01M 4/38, H01G 11/02, C07C 317/06, C01F 5/00

(54) **ELECTRODE, METHOD FOR MANUFACTURING SAME, AND ELECTROCHEMICAL DEVICE**
ELEKTRODE, VERFAHREN ZUR HERSTELLUNG DAVON UND ELEKTROCHEMISCHE VORRICHTUNG
ÉLECTRODE, PROCÉDÉ DE FABRICATION DE CELLE-CI, ET DISPOSITIF ÉLECTROCHIMIQUE

(30) Priority: 06.02.2015 JP 2015022297
(43) Date of publication of application: 13.12.2017
(73) Proprietor: Murata Manufacturing Co., Ltd., Nagaokakyo-shi, Kyoto 617-8555 (JP)
(72) Inventor: NAKAYAMA, Yuri, Tokyo 108-0075 (JP); MATSUMOTO, Ryuhei, Tokyo 108-0075 (JP); KAWASAKI, Hideki, Kyoto-shi Kyoto 600-8813 (JP)
(74) Representative: Witte, Weller & Partner Patentanwälte mbB
(86) International application number: PCT/JP2015/006166
(87) International publication number: WO 2016/125217

(56) References cited:
- EP-A1- 2 713 431
- EP-A2- 2 472 647
- WO-A1-2016/006299
- CN-A- 101 997 107
- JP-A- 2003 100 347
- JP-A- 2012 531 725
- JP-A- 2014 072 031
- JP-A- 2015 065 028
- KR-A- 20140 138 476
- US-A1- 2011 159 381

## Description

### TECHNICAL FIELD

The present technology relates to a magnesium-containing electrode, a method for production thereof, and an electrochemical device.

### BACKGROUND ART

Magnesium is a resource more abundant and much more inexpensive than lithium. Magnesium is capable of producing a large amount of electricity per unit volume through an oxidation-reduction reaction as compared with lithium, and also has high safety in a case where being used in batteries. Therefore, magnesium-ion batteries are attracting attention as next-generation secondary batteries to replace lithium-ion batteries.

Magnesium, which can form an oxide and various passive films, often has an electrochemically inert surface state (see, for example, Non-Patent Document 1). Such an inert surface state can cause a large overpotential during the dissolution and precipitation reaction of Mg. Thus, there has not been to date any commercialized secondary battery using a magnesium metal negative electrode. US 2011/159381 A1 discloses a magnesium battery electrode assembly including a current collector comprising a carbonaceous material and an electrode layer comprising an electrode active material disposed on the current collector. EP 2 713 431 A1 discloses an electrolyte solution including a solvent formed from a sulfone, and a magnesium salt dissolved in the solvent. KR 2014 0138476 A discloses an anode foil for a magnesium secondary battery.

### CITATION LIST

### NON-PATENT DOCUMENT

Non-Patent Document 1: J. Electro analytical Chem. 466, 203 (1999)

### SUMMARY OF THE INVENTION

### PROBLEMS TO BE SOLVED BY THE INVENTION

It is an object of the present technology to provide an electrochemically active electrode, a method for producing such an electrode, and an electrochemical device.

### SOLUTIONS TO PROBLEMS

The present invention provides for an electrode comprising an active material layer according to claim 1, an electrochemical device according to claim 6 and an electrode producing method according to claim 7. Embodiments of the invention are set forth with the dependent claims.

To solve the above problems, a first technology is directed to an electrode comprising an active material layer formed by electrochemical magnesium plating using an electrolytic solution including a sulfone and a magnesium salt dissolved in the sulfone, thereby said active material layer including at least magnesium, carbon, oxygen, sulfur, and halogen and having a surface exhibiting a single peak derived from magnesium in the range of 40 eV to 60 eV.

A second technology is directed to an electrochemical device including a positive electrode, a negative electrode, and an electrolyte, in which the negative electrode comprises an active material layer formed by electrochemical magnesium plating using an electrolytic solution including a sulfone and a magnesium salt dissolved in the sulfone, thereby said active material layer includes at least magnesium, carbon, oxygen, sulfur, and halogen and has a surface exhibiting a single peak derived from magnesium in the range of 40 eV to 60 eV.

A third technology is directed to an electrode producing method including performing electrochemical magnesium plating using an electrolytic solution including a sulfone and a magnesium salt to provide an active material layer comprised by the electrode, wherein said active material layer comprises at least magnesium, carbon, oxygen, sulfur, and halogen and has a surface exhibiting a single peak derived from magnesium in a range of 40 eV to 60 eV.

According to an aspect an electrode is obtainable by electrochemical plating using an electrolytic solution including a sulfone and a magnesium salt.

### EFFECTS OF THE INVENTION

As described above, the present technology makes it possible to provide an electrochemically active electrode.

### BRIEF DESCRIPTION OF DRAWINGS

Fig. 1 is a schematic diagram illustrating an example of the structure of a magnesium-ion battery according to a first embodiment of the present technology.
Figs. 2(a) to 2(e) are graphs showing the XPS spectrum of a plated Mg layer of Example 1.
Fig. 3 is a graph showing the depth direction dependence of the XPS spectrum of the plated Mg layer of Example 1.
Fig. 4 is a graph showing the CV spectra of Mg electrodes of Example 1 and Comparative Example 2.
Fig. 5 is a graph showing the CV spectra of Mg electrodes of Example 2 and Comparative Example 2.
Fig. 6 is a graph showing the CV spectra of Mg electrodes of Example 3 and Comparative Example 2.
Figs. 7(a) to 7(c) are graphs showing the XPS spectrum of a plated Mg layer of Comparative Example 1.
Fig. 8 is a graph showing the result of waveform separation of the XPS spectrum of the plated Mg layer of Comparative Example 1.
Figs. 9(a) to 9(e) are graphs showing the XPS spectrum of a plated Mg layer of Comparative Example 2.
Fig. 10 is a graph showing the depth direction dependence of the XPS spectrum of the plated Mg layer of Comparative Example 2.
Fig. 11(a) is a graph showing the result of waveform separation of the XPS spectrum of the surface of the plated Mg layer of Comparative Example 2. Fig. 11(b) is a graph showing the result of waveform separation of the XPS spectrum of a portion exposed by etching the plated Mg layer of Comparative Example 2 from the surface to a depth of about 100 nm. Fig. 11(c) is a graph showing the result of waveform separation of the XPS spectrum of a portion exposed by etching the plated Mg layer of Comparative Example 2 from the surface to a depth of about 200 nm.
Fig. 12 is an exploded perspective view illustrating the structure of a magnesium-sulfur secondary cell of Example 4.
Fig. 13 is a graph showing the charge-discharge curve of the magnesium-sulfur secondary cell of Example 4.

### MODE FOR CARRYING OUT THE INVENTION

Basically, the electrochemical device may be of any type. Specific examples of the electrochemical device include various batteries, capacitors, and sensors with electrodes including magnesium, and magnesium ion filters. Batteries with electrodes including magnesium are, for example, secondary batteries, air cells, and fuel cells.

The electrochemical device may be installed in or used to supply power to drive power sources or auxiliary power sources for notebook personal computers, personal digital assistants (PDAs), cellular phones, base and extension units for cordless phones, video cameras, digital still cameras, digital books, electronic dictionaries, portable music players, radios, headphones, game machines, navigation systems, memory cards, cardiac pacemakers, hearing aids, electric tools, electric shavers, refrigerators, air conditioners, televisions, stereos, water heaters, microwave ovens, dishwashers, washing machines, drying machines, lighting devices, toys, medical instruments, robots, road conditioners, traffic signals, railway vehicles, golf carts, electric carts, and electric cars (including hybrid cars). The electrochemical device may also be installed in or used to supply power to power storage sources for buildings such as houses or power generation facilities. The electrochemical device may also be used as an electrical storage device in what are called smart grids. Such an electrical storage device can not only supply power but also store power by receiving power from other power sources. Examples of other power sources that can be used include thermal power generators, nuclear power generators, hydroelectric power generators, solar batteries, wind power generators, geothermal power generators, and fuel cells (including biofuel cells).

Embodiments of the present technology will be described in the following order.
1 First Embodiment (electrode and method for production thereof)
2 Second Embodiment (magnesium-ion battery)

### <1 First embodiment>

### [Configurations of electrode]

An electrode according to a first embodiment of the present technology includes a collector and an active material layer provided on the surface of the collector. The electrode is what is called a magnesium electrode, which is suitable for use as an electrode for magnesium-ion batteries.

### (Collector)

The collector includes a metal foil such as a copper foil, a nickel foil, or a stainless steel foil.

### (Active material layer)

The active material layer is a layer having magnesium ion conductivity and containing at least magnesium (Mg), carbon (C), oxygen (O), sulfur (S), and halogen at and near its surface. In addition, the active material layer has a surface exhibiting a single peak derived from magnesium in the range of 40 eV to 60 eV. The halogen may be, for example, at least one selected from the group consisting of fluorine (F), chlorine (Cl), bromine (Br), and iodine (I).

The active material layer preferably exhibits, over a region from its surface to a depth of 200 nm, a single peak derived from magnesium in the range of 40 eV to 60 eV. This is because in such a case, the active material layer will be electrochemically active over the region from the surface to the inside. In addition, for a similar reason, the oxidized state of magnesium is preferably substantially constant from the surface to a depth of 200 nm in the active material layer.

The active material layer preferably exhibits, over the depth from its front surface to its back surface, a single peak derived from magnesium in the range of 40 eV to 60 eV. This is because in such a case, the whole of the active material layer will have good electrical activity. In addition, for a similar reason, the oxidized state of magnesium in the active material layer is preferably substantially constant from the front surface to the back surface of the active material layer. In this context, the term "the front surface of the active material layer" means one of the two surfaces of the active material layer, on the side where the electrode surface is formed, and the term "the back surface of the active material layer" means the other surface opposite to the front surface, in other words, the other surface on the side where the collector-active material layer interface is formed.

Whether the active material layer contains the elements mentioned above can be determined by analyzing the active material layer using X-ray photoelectron spectroscopy (XPS). In addition, XPS may also be used to determine whether the active material layer exhibits the peak mentioned above and whether the oxidized state of magnesium is as mentioned above in the active material layer.

The active material layer is preferably a plated layer, which can be formed by electrochemical plating using an electrolytic solution including a sulfone and a magnesium salt dissolved in the sulfone. Note that the sulfone and the magnesium salt will be described below in the section "Electrode producing method."

### [Electrode producing method]

Next, an example of an electrode producing method will be described.

First, a magnesium ion-containing nonaqueous electrolytic solution is prepared, including a solvent including a sulfone and a magnesium salt dissolved in the solvent. In the electrolytic solution, the molar ratio of the sulfone to the magnesium salt is, for example, but not limited to, 4 to 35, typically 6 to 16, preferably 7 to 9.

The sulfone is, for example, an alkyl sulfone represented by R₁R₂SO₂, in which R₁ and R₂ each represent an alkyl group, or an alkyl sulfone derivative. In this regard, the type of the R₁ and R₂ groups (the number of carbon atoms in the R₁ and R₂ groups and the combination of the R₁ and R₂ groups) is not limited and may be selected as needed. The number of carbon atoms in each of the R₁ and R₂ groups is preferably, but not limited to, 4 or less. In addition, the sum of the numbers of carbon atoms in the R₁ and R₂ groups is preferably, but not limited to, 4 to 7. R₁ and R₂ are each, for example, methyl, ethyl, n-propyl, isopropyl, n-butyl, isobutyl, sec-butyl, or tert-butyl. Specifically, the alkyl sulfone is at least one selected from the group consisting of dimethyl sulfone (DMS), methyl ethyl sulfone (MES), methyl n-propyl sulfone (MnPS), methyl isopropyl sulfone (MiPS), methyl n-butyl sulfone (MnBS), methyl isobutyl sulfone (MiBS), methyl sec-butyl sulfone (MsBS), methyl tert-butyl sulfone (MtBS), ethyl methyl sulfone (EMS), diethyl sulfone (DES), ethyl n-propyl sulfone (EnPS), ethyl isopropyl sulfone (EiPS), ethyl n-butyl sulfone (EnBS), ethyl isobutyl sulfone (EiBS), ethyl sec-butyl sulfone (EsBS), ethyl tert-butyl sulfone (EtBS), di-n-propyl sulfone (DnPS), diisopropyl sulfone (DiPS), n-propyl n-butyl sulfone (nPnBS), n-butyl ethyl sulfone (nBES), isobutyl ethyl sulfone (iBES), sec-butyl ethyl sulfone (sBES), and di-n-butyl sulfone (DnBS). The alkyl sulfone derivative is, for example, ethyl phenyl sulfone (EPhS).

Among the above sulfones, at least one selected from the group consisting of EnPS, EiPS, EsBS, and DnPS is preferred. This is because EnPS, EiPS, EsBS, and DnPS, which can form a magnesium (Mg) complex having a six-coordinated monomer structure in the electrolytic solution, have a high ability to supply Cl⁻ (chloride anion) to the electrolytic solution and is capable of stably forming an active magnesium complex. This is also because among the above sulfones, EnPS, EiPS, EsBS, and DnPS can stably form an active Mg complex having a four-coordinated dimer structure in the electrolytic solution.

The magnesium salt includes, for example, at least one selected from the group consisting of magnesium chloride (MgCl₂), magnesium bromide (MgBr₂), magnesium iodide (MgI₂), magnesium perchlorate (Mg(ClO₄)₂), magnesium tetrafluoroborate (Mg(BF₄)₂), magnesium hexafluorophosphate (Mg(PF₆)₂), magnesium hexafluoroarsenate (Mg(AsF₆)₂), magnesium perfluoroalkylsulfonate (Mg(Rf1SO₃)₂, Rf1 is a perfluoroalkyl group), and magnesium perfluoroalkylsulfonylimidate (Mg((Rf2SO₂)₂N)₂, Rf2 is a perfluoroalkyl group). Among these magnesium salts, MgX₂ (X = Cl, Br, I) is particularly preferred.

If necessary, the electrolytic solution may further contain an additive. The additive may be, for example, a salt including a metal ion or cation of at least one atom or atomic group selected from the group consisting of aluminum (Al), beryllium (Be), boron (B), gallium (Ga), indium (In), silicon (Si), tin (Sn), titanium (Ti), chromium (Cr), iron (Fe), cobalt (Co), and lanthanum (La). Alternatively, the additive may be a salt including at least one atom, organic group, or anion selected from the group consisting of hydrogen, an alkyl group, an alkenyl group, an aryl group, a benzyl group, an amide group, a fluoride ion (F⁻), a chloride ion (Cl⁻), a bromide ion (Br⁻), an iodide ion (I⁻), a perchlorate ion (ClO₄⁻), a tetrafluoroborate ion (BF₄⁻), a hexafluorophosphate ion (PF₆⁻), a hexafluoroarsenate ion (AsF₆⁻), a perfluoroalkylsulfonate ion (Rf1SO₃⁻, Rf1 is a perfluoroalkyl group), and a perfluoroalkylsulfonylimide ion ((Rf2SO₂)₂N⁻, Rf2 is a perfluoroalkyl group) . The addition of the additive can improve the ionic conductivity of the electrolytic solution.

Subsequently, using the magnesium ion-containing nonaqueous electrolytic solution, a plated layer is formed as an active material layer by performing electrochemical plating to deposit Mg metal on the metal foil.

As a result, the desired electrode is obtained.

### [Advantageous effects]

The active material layer of the electrode according to the first embodiment includes at least magnesium (Mg), carbon (C), oxygen (0), sulfur (S), and halogen and has a surface exhibiting a single peak derived from magnesium in the range of 40 eV to 60 eV. Surface oxidation and surface passive film formation are suppressed on such a surface. Therefore, the surface of the active material layer has good electrochemical activity.

In the electrode producing method according to the first embodiment, an active material layer is formed by electrochemical plating using an electrolytic solution containing a magnesium salt dissolved in a solvent including a sulfone. Therefore, the resulting active material layer has an electrochemically active surface. In addition, the use of the electrochemical magnesium plating technology makes it possible to obtain a thin electrode.

The use of the electrochemical magnesium plating technology makes it possible to reduce the electrode production costs. In general, metal thin films are formed by rolling. However, Mg thin film formation requires rolling to be performed over and over again because of its plastic properties. Therefore, rolling a magnesium material to form an electrode will lead to an increase in electrode production cost.

### [Modifications]

### (Modification 1)

The electrochemical plating may also be performed using a magnesium ion-containing nonaqueous electrolytic solution including: a solvent including a sulfone and a nonpolar solvent; and a magnesium salt dissolved in the solvent. In the electrolytic solution, the molar ratio of the sulfone to the magnesium salt is, for example, but not limited to, 4 to 20, typically 6 to 16, preferably 7 to 9.

The nonpolar solvent is preferably a nonaqueous solvent with a dielectric constant of 20 or less and a donor number of 20 or less. More specifically, the nonpolar solvent is, for example, at least one selected from the group consisting of an aromatic hydrocarbon, an ether, a ketone, an ester, and a chain carbonate. The aromatic hydrocarbon may be, for example, toluene, benzene, o-xylene, m-xylene, p-xylene, or 1-methylnaphthalene. The ether may be, for example, diethyl ether or tetrahydrofuran. The ketone may be, for example, 4-methyl-2-pentanone. The ester may be, for example, methyl acetate or ethyl acetate. The chain carbonate may be, for example, dimethyl carbonate, diethyl carbonate, or ethylmethyl carbonate.

### (Modification 2)

The above embodiment has been described with reference to an example where the electrode includes a collector and an active material layer provided on the surface of the collector. However, the structure of the electrode is not limited to this structure. For example, the electrode may have a structure including only the active material layer.

### <2 Second Embodiment>

A second embodiment of the present technology will be described, providing a magnesium-ion battery as an example of an electrochemical device having, as a negative electrode, the electrode according the first embodiment.

### [Configurations of magnesium-ion battery]

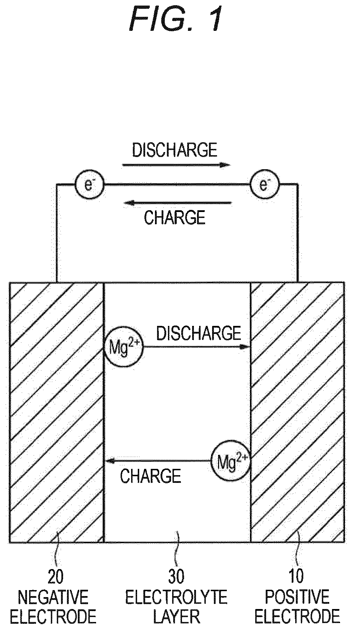

As illustrated in Fig. 1, a magnesium-ion battery according to the first embodiment of the present technology includes a positive electrode 10, a negative electrode 20, and an electrolyte layer 30. If necessary, the magnesium-ion battery may further include a separator placed between the positive electrode 10 and the negative electrode 20. In this case, the separator is impregnated with an electrolytic solution, which is contained in the electrolyte layer 30.

### (Positive electrode)

The positive electrode 10 has, for example, a structure including a positive electrode collector and a positive electrode active material layer provided on the surface of the positive electrode collector. Alternatively, the positive electrode 10 may also have a structure including only a positive electrode active material layer with no positive electrode collector. The positive electrode collector includes, for example, a metal foil such as an aluminum foil. For example, the positive electrode active material layer includes, as a positive electrode active material, sulfur (S), graphite fluoride ((CF)n), or an oxide or halide of any of various metals (such as scandium (Sc), titanium (Ti), vanadium (V), chromium (Cr), manganese (Mn), iron (Fe), cobalt (Co), nickel (Ni), copper (Cu), and zinc (Zn)).

If necessary, the positive electrode active material layer may contain at least one of a conductive aid and a binder. Examples of the conductive aid include carbon materials such as graphite, carbon fibers, carbon black, and carbon nanotubes. One of these materials or a mixture of two or more of these materials may be used. Examples of carbon fibers that may be used include vapor growth carbon fibers (VGCFs). Examples of carbon black that may be used include acetylene black and ketjen black. Examples of carbon nanotubes that may be used include single-wall carbon nanotubes (SWCNTs) and multi-wall carbon nanotubes (MWCNTs) such as double-wall carbon nanotubes (DWCNTs). In addition, if having good conductivity, other materials than carbon materials may also be used, such as metal materials such as Ni powders and conductive polymer materials.

Examples of the binder that may be used include fluororesins such as polyvinylidene fluoride (PVdF) and polytetrafluoroethylene (PTFE), polyvinyl alcohol (PVA) resins, styrene-butadiene copolymer rubber (SBR) resins, and other polymer resins. The binder may also include a conductive polymer. Examples of the conductive polymer that may be used include substituted or unsubstituted polyaniline, polypyrrole, and polythiophene, and (co)polymers including one or two of these polymers.

### (Negative electrode)

The negative electrode 20 may be the electrode according to the first embodiment or the electrode according to the modified examples thereof.

### (Electrolyte layer)

The electrolyte layer 30 includes an electrolytic solution. The electrolytic solution includes a solvent and an electrolyte salt, in which the electrolyte salt is dissolved in the solvent.

The electrolyte salt may be, for example, a magnesium salt. A single electrolyte salt or a mixture of two or more electrolyte salts may be used. Examples of the magnesium salt that may be used include those for the electrolytic solution in the first embodiment.

Examples of the solvent include a sulfone, tetrahydrofuran, ethylene carbonate, propylene carbonate, dimethyl carbonate, diethyl carbonate, ethylmethyl carbonate, methylpropyl carbonate, acetonitrile, dimethoxyethane, diethoxyethane, vinylene carbonate, and γ-butyrolactone. These solvents may be used alone, or a mixture of two or more of these solvents may be used. Examples of the sulfone that may be used include those for the electrolytic solution in the first embodiment.

### (Separator)

The separator is provided to separate the positive and negative electrodes 10 and 20 from each other and prevent both electrodes from coming into contact with each other and causing a short-circuit current, and is configured to allow magnesium ions passing through it. The separator may be, for example, any of an inorganic separator and an organic separator.

The inorganic separator may be, for example, a glass filter. Examples of the organic separator that may be used include porous membranes made of a synthetic resin such as polytetrafluoroethylene, polypropylene, or polyethylene. A laminated structure of two or more of these porous membranes may also be used. In particular, porous membranes made of polyolefin are preferred because they are highly effective in preventing short circuits and can produce a shut-down effect to improve battery safety.

### [Operation of magnesium-ion battery]

During charging, the magnesium-ion battery having the configurations described above stores electricity by converting electrical energy to chemical energy through movement of magnesium ions (Mg²⁺) from the positive electrode 10 to the negative electrode 20 through the electrolyte layer 30. During discharging, the magnesium-ion battery generates electrical energy through movement of magnesium ions from the negative electrode 20 back to the positive electrode 10 through the electrolyte layer 30.

### [Advantageous effects]

The magnesium-ion battery according to the second embodiment is designed to have the electrode according to the first embodiment as a negative electrode, which makes is possible to provide a magnesium-ion battery having good charge-discharge characteristics. This also makes it possible to provide a magnesium-ion battery with high energy density.

### [Modifications]

The electrolyte layer 30 may include an electrolytic solution and a polymer compound that acts as a retainer to hold the electrolytic solution, in which the polymer compound is allowed to swell with the electrolytic solution. In this case, the polymer compound allowed to swell with the electrolytic solution may be in the form of a gel.

The polymer compound may be, for example, polyacrylonitrile, polyvinylidene fluoride, a vinylidene fluoride-hexafluoropropylene copolymer, polytetrafluoroethylene, polyhexafluoropropylene, polyethylene oxide, polypropylene oxide, polyphosphazen, polysiloxane, polyvinyl acetate, polyvinyl alcohol, polymethyl methacrylate, polyacrylic acid, polymethacrylic acid, styrene-butadiene rubber, nitrile-butadiene rubber, polystyrene, or polycarbonate. Polyacrylonitrile, polyvinylidene fluoride, polyhexafluoropropylene, or polyethylene oxide is particularly preferred in view of electrochemical stability. The electrolyte layer 30 may also be a solid electrolyte layer.

### Examples

Hereinafter, the present technology will be more specifically described with reference to examples. It will be understood that the examples are not intended to limit the present technology in any way.

### (Example 1)

First, a Mg ion-containing electrolytic solution was prepared containing MgCl₂ and ethyl n-propyl sulfone (EnPS) in a ratio of MgCl₂ to EnPS of 1/8 (molar ratio). Subsequently, using the Mg ion-containing electrolytic solution, a plated Mg layer was formed as an active material layer on a Cu foil by electrochemical plating to deposit Mg metal on the Cu foil. As a result, the desired Mg electrode was obtained.

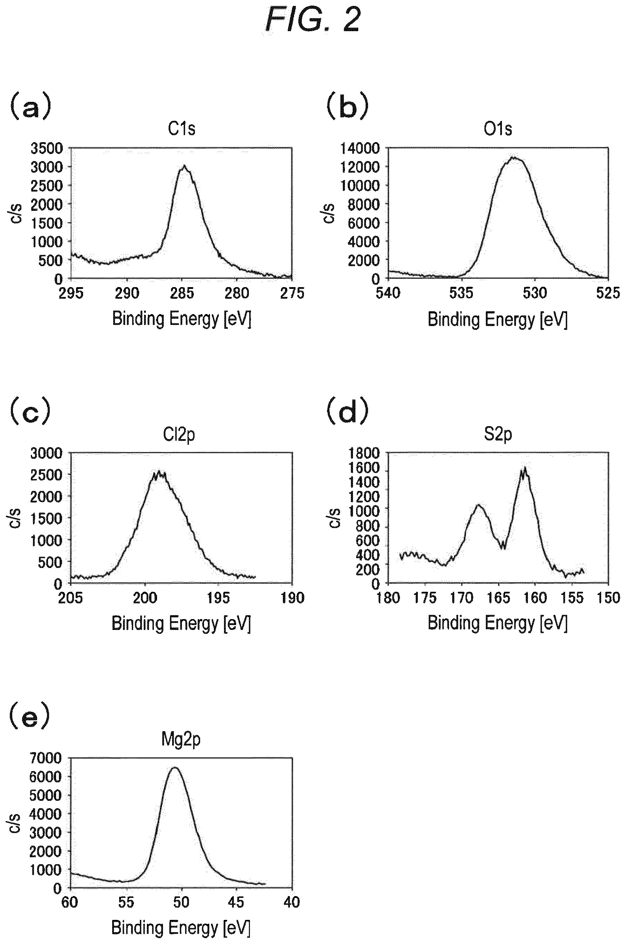

Subsequently, the surface of the plated Mg layer obtained by the electrochemical plating was analyzed by XPS. As a result, it was found that Mg, C, O, S, and Cl were present at the surface of the plated Mg layer (see Figs. 2(a) to 2(e)). Additionally, in the surface analysis, a single peak derived from Mg was observed in the range of 40 eV to 60 eV with no splitting of the Mg-derived peak.

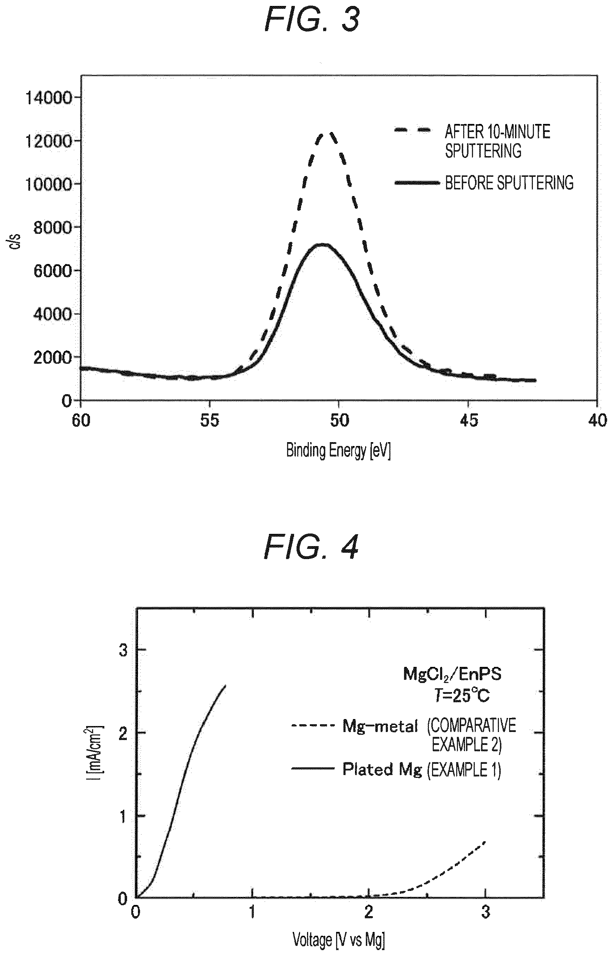

Subsequently, the plated Mg layer was etched from the surface to a depth of about 200 nm by Ar sputtering, and the resulting surface was analyzed by XPS. As a result, it was found that the position and shape of the Mg-derived peak did not change before and after the Ar sputtering (see Fig. 3).

A three-electrode cell was also prepared using, as a working electrode, the Mg electrode obtained by the electrochemical plating, using surface-polished Mg metal foils as counter and reference electrodes, and using the Mg ion-containing electrolytic solution. The resulting cell was subjected to I-V measurement, in which dissolution of Mg was observed with no overpotential (see Fig. 4) .

### (Example 2)

A Mg electrode was obtained as in Example 1, except that a Mg ion-containing electrolytic solution containing MgBr₂ and ethyl n-propyl sulfone (EnPS) in a ratio of MgBr₂ to EnPS of 1/8 (molar ratio) was used instead.

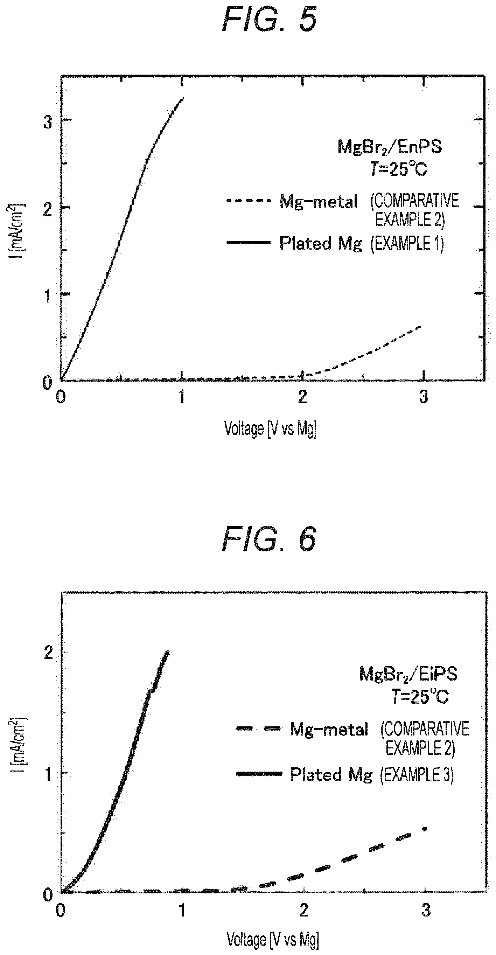

Subsequently, I-V measurement was performed as in Example 1, except that the Mg electrode obtained by the electrochemical plating shown above was used as the working electrode. As a result, dissolution of Mg was observed with no overpotential (see Fig. 5).

### (Example 3)

First, a Mg electrode was obtained as in Example 1, except that a Mg ion-containing electrolytic solution containing MgBr₂ and ethyl isopropyl sulfone (EiPS) in a ratio of MgBr₂ to EiPS of 1/8 (molar ratio) was used instead.

Subsequently, I-V measurement was performed as in Example 1, except that the Mg electrode obtained by the electrochemical plating shown above was used as the working electrode. As a result, dissolution of Mg was observed with no overpotential (see Fig. 6).

### (Comparative Example 1)

First, a commercially available Mg metal foil (manufactured by The Nilaco Corporation) was provided as a Mg electrode.

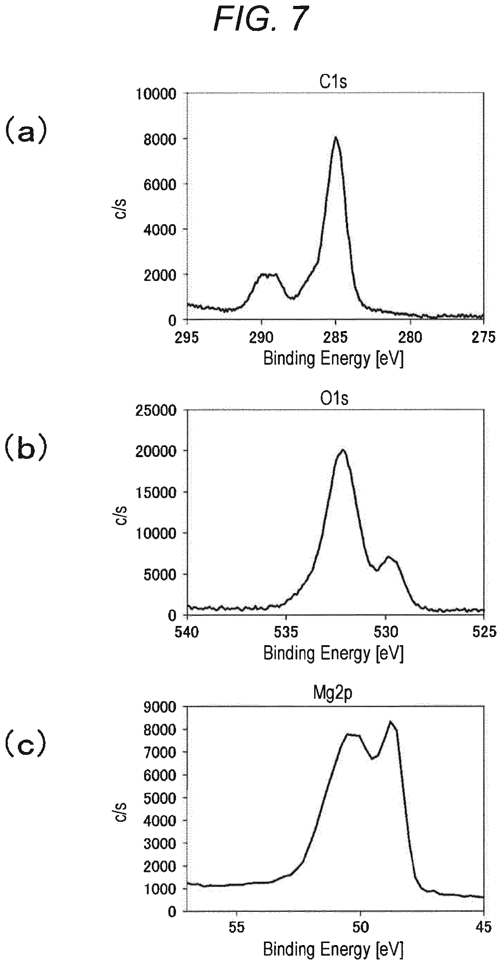

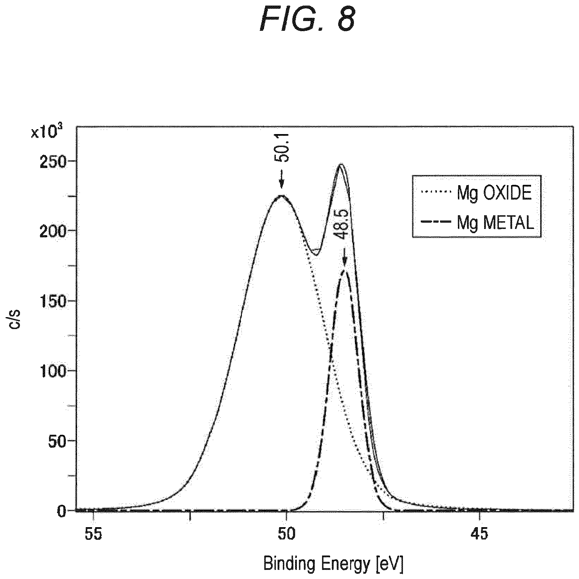

Subsequently, the surface of the Mg electrode provided was analyzed by XPS. As a result, it was observed that Mg, C, and O were present at the surface of the Mg electrode (see Figs. 7(a) to 7(c)). Additionally, in the surface analysis, the Mg-derived peak was observed to split into two components, which showed the presence of oxidized Mg. The ratio of Mg oxide to Mg metal was estimated to be 21 mol% (48.5 eV) : 79 mol% (50.1 eV) from the area ratio (see Fig. 8).

### (Comparative Example 2)

First, a commercially available Mg metal foil was immersed overnight in a Mg electrolytic solution. Subsequently, the Mg metal foil was washed with ethyl n-propyl sulfone (EnPS) and toluene and then dried in an Ar glove box to give a Mg electrode.

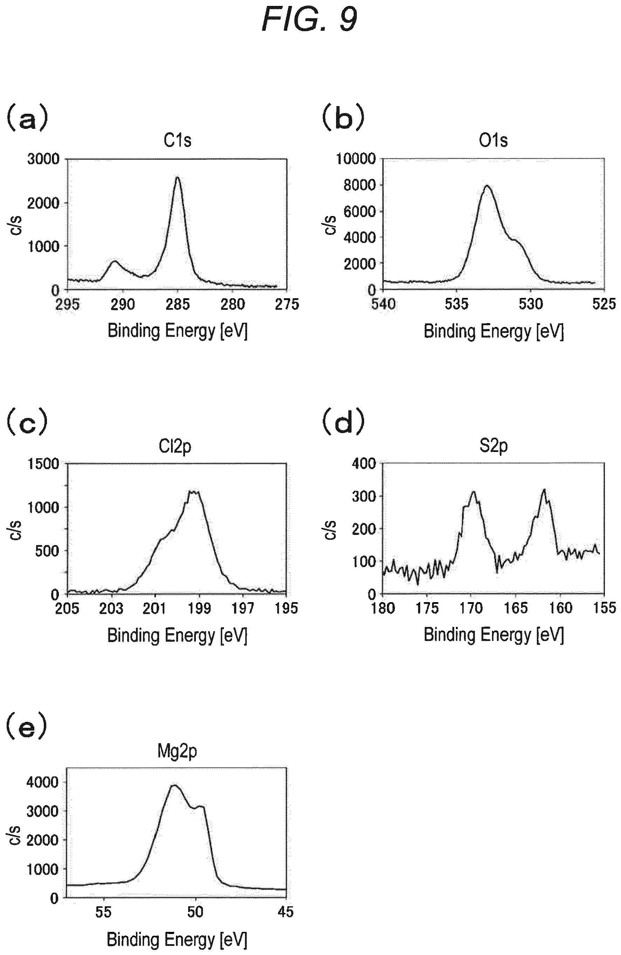

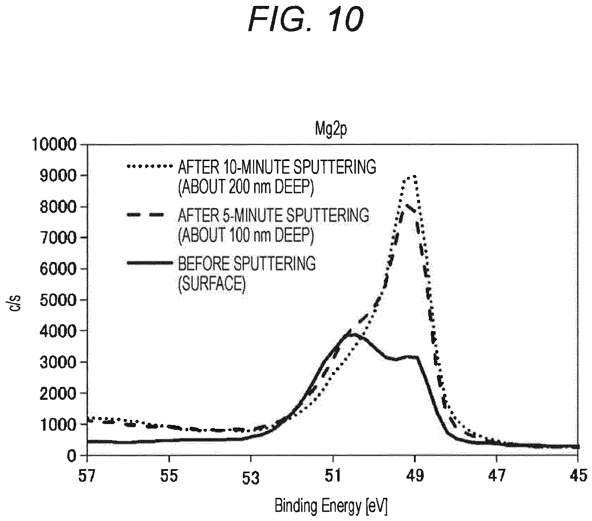

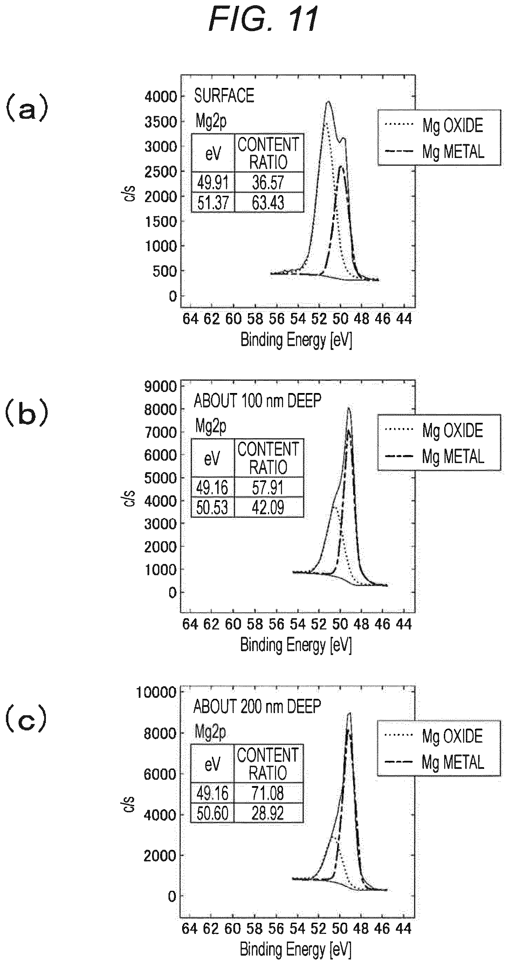

Subsequently, the surface of the Mg electrode obtained after the immersion in the Mg electrolytic solution was analyzed by XPS. As a result, it was found that Mg, C, O, S, and Cl were present at the surface of the Mg electrode (see Figs. 9 (a) to 9(e)). Additionally, in the surface analysis, the Mg-derived peak was observed to split into two components, and the ratio of Mg oxide to Mg metal was estimated to be 63 mol% : 37 mol% from the area ratio (see Figs. 10 and 11(a)).

Subsequently, the Mg electrode was etched from the surface to a depth of about 100 nm and then to a depth of about 200 nm by Ar sputtering while the surface at each of these positions was analyzed by XPS. As a result, it was found that the oxide content decreased with increasing depth from the surface of the Mg electrode (see Figs. 10 and 11(a) to 11(c)).

I-V measurement was also performed as in Example 1, except that the Mg electrode obtained after the immersion in the Mg electrolytic solution was used instead. As a result, an overpotential of at least 2 V was required for the dissolution of Mg (see Figs. 4 to 6).

The results have shown the following.

Electrochemical Mg plating using an electrolytic solution containing a sulfone and a Mg salt makes it possible to obtain a Mg electrode having a surface exhibiting a single peak derived from magnesium in the range of 40 eV to 60 eV, in other words, a Mg electrode having an electrochemically active surface.

Electrochemical Mg plating using an electrolytic solution containing a sulfone and a Mg salt also makes it possible to obtain a Mg electrode in which the oxidized state of Mg is substantially constant from its surface to a depth of 200 nm, in other words, a Mg electrode also having an electrochemically active inner portion.

### (Example 4)

A coin-type, magnesium-sulfur secondary cell (hereinafter referred to as the "coin cell") was prepared using, as a negative electrode, the Mg electrode prepared by the electrochemical plating in Example 1, using, as a positive electrode, a mixture of sulfur, a conductive aid, and a binder, and using a Mg electrolytic solution containing MgCl₂ and ethyl n-propyl sulfone (EnPS) in a ratio of MgCl₂ to EnPS of 1/8 (molar ratio).

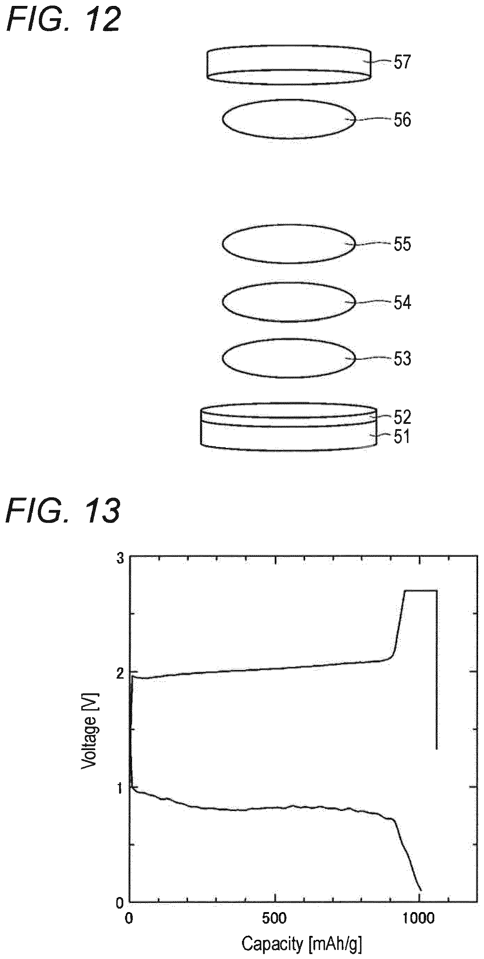

As illustrated in Fig. 12, the coin cell was prepared by placing a gasket 52 on a coin cell can 51, stacking the positive electrode 53 of the mixture, a separator 54 made of a glass filter, the negative electrode 55 made of the Mg electrode of Example 1, a spacer 56 made of a 500-µm-thick stainless steel sheet, and a coin cell lid 57 in this order, and crimping the coin cell can 51 to seal it. The spacer 56 was spot-welded to the coin cell lid 57 in advance.

When the characteristics of the cell were evaluated, a reversible charge-discharge reaction was observed (see Fig. 13). The reason for allowing such a charge-discharge reaction is that surface oxidation and passive film formation are suppressed on the Mg electrode prepared by the electrochemical plating so that the active material surface has good electrochemical activity.

Embodiments of the present technology and modifications thereof, and examples of the present technology have been described specifically. It will be understood that the embodiments, the modifications, and the examples described above are not intended to limit the present technology and that they may be altered or modified in various manners that fall within the scope of the appended claims.

For example, the configurations, methods, processes, shapes, materials, values, and other conditions shown in the embodiments, the modifications thereof, and the examples are only by way of example, and if necessary, configurations, methods, processes, shapes, materials, values, and other conditions different from the above may also be used.

## Claims

1. An electrode comprising an active material layer formed by electrochemical magnesium plating using an electrolytic solution including a sulfone and a magnesium salt dissolved in the sulfone, thereby said active material layer comprising at least magnesium, carbon, oxygen, sulfur, and halogen and having a surface exhibiting a single peak derived from magnesium in a range of 40 eV to 60 eV.

2. The electrode according to claim 1, which exhibits, over a region from the surface to a depth of 200 nm, a single peak derived from magnesium in a range of 40 eV to 60 eV.

3. The electrode according to claim 1, wherein an oxidized state of magnesium is substantially constant from the surface to a depth of 200 nm.

4. The electrode according to claim 1, further comprising a collector and the active material layer provided on the collector, wherein the active material layer includes at least magnesium, carbon, oxygen, sulfur, and halogen and exhibits, over a depth from one to another surface of the active material layer, a single peak derived from magnesium in a range of 40 eV to 60 eV.

5. The electrode according to claim 1, further comprising a collector and the active material layer provided on the collector, wherein the active material layer includes at least magnesium, carbon, oxygen, sulfur, and halogen, and an oxidized state of magnesium is substantially constant from one to another surface of the active material layer.

6. An electrochemical device comprising a positive electrode (10), a negative electrode (20), and an electrolyte (30), wherein the negative electrode (20) comprises an active material layer formed by electrochemical magnesium plating using an electrolytic solution including a sulfone and a magnesium salt dissolved in the sulfone, thereby said active material layer includes at least magnesium, carbon, oxygen, sulfur, and halogen and has a surface exhibiting a single peak derived from magnesium in a range of 40 eV to 60 eV.

7. An electrode producing method comprising performing electrochemical magnesium plating using an electrolytic solution including a sulfone and a magnesium salt to provide an active material layer comprised by the electrode, wherein said active material layer comprises at least magnesium, carbon, oxygen, sulfur, and halogen and has a surface exhibiting a single peak derived from magnesium in a range of 40 eV to 60 eV.

8. The electrode producing method according to claim 7, wherein the sulfone comprises at least one selected from the group consisting of ethyl isopropyl sulfone, ethyl n-propyl sulfone, ethyl sec-butyl sulfone, and di-n-propyl sulfone.

9. The electrode producing method according to claim 7, wherein the magnesium salt comprises at least one selected from the group consisting of magnesium chloride, magnesium bromide, magnesium iodide, magnesium perchlorate, magnesium tetrafluoroborate, magnesium hexafluorophosphate, magnesium hexafluoroarsenate, magnesium perfluoroalkylsulfonate, and magnesium perfluoroalkylsulfonylimidate.

## Patentansprüche

1. Elektrode, mit einer Schicht aus aktivem Material, die durch elektrochemische Magnesiumplattierung unter Verwendung einer elektrolytischen Lösung, die ein Sulfon und ein in dem Sulfon gelöstes Magnesiumsalz aufweist, gebildet wird, wobei die Schicht aus aktivem Material mindestens Magnesium, Kohlenstoff, Sauerstoff, Schwefel und Halogen aufweist und eine Oberfläche aufweist, die einen einzelnen, von Magnesium abgeleiteten Peak bzw. Spitzenwert in einem Bereich von 40 eV bis 60 eV aufweist.

2. Elektrode nach Anspruch 1, die über einen Bereich von der Oberfläche bis zu einer Tiefe von 200 nm einen einzelnen, von Magnesium abgeleiteten Peak bzw. Spitzenwert in einem Bereich von 40 eV bis 60 eV aufweist.

3. Elektrode nach Anspruch 1, wobei ein oxidierter Zustand des Magnesiums von der Oberfläche bis zu einer Tiefe von 200 nm im Wesentlichen konstant ist.

4. Elektrode nach Anspruch 1, die des Weiteren einen Kollektor und die auf dem Kollektor vorgesehene Schicht aus aktivem Material aufweist, wobei die Schicht aus aktivem Material mindestens Magnesium, Kohlenstoff, Sauerstoff, Schwefel und Halogen aufweist und über eine Tiefe von einer zu einer anderen Oberfläche der Schicht aus aktivem Material einen einzelnen, von Magnesium abgeleiteten Peak bzw. Spitzenwert in einem Bereich von 40 eV bis 60 eV aufweist.

5. Elektrode nach Anspruch 1, die des Weiteren einen Kollektor und die auf dem Kollektor vorgesehene aktive Materialschicht aufweist, wobei die aktive Materialschicht mindestens Magnesium, Kohlenstoff, Sauerstoff, Schwefel und Halogen aufweist und ein oxidierter Zustand des Magnesiums von einer zur anderen Oberfläche der aktiven Materialschicht im Wesentlichen konstant ist.

6. Elektrochemische Vorrichtung, die eine positive Elektrode (10), eine negative Elektrode (20) und ein Elektrolyt (30) aufweist, wobei die negative Elektrode (20) eine Schicht aus aktivem Material aufweist, die durch elektrochemische Magnesiumplattierung unter Verwendung einer elektrolytischen Lösung, die ein Sulfon und ein in dem Sulfon gelöstes Magnesiumsalz aufweist, gebildet wird, wobei die Schicht aus aktivem Material mindestens Magnesium, Kohlenstoff, Sauerstoff, Schwefel und Halogen aufweist und eine Oberfläche aufweist, die einen einzelnen, von Magnesium abgeleiteten Peak bzw. Spitzenwert in einem Bereich von 40 eV bis 60 eV aufweist.

7. Verfahren zur Herstellung einer Elektrode, mit dem Durchführen einer elektrochemischen Magnesiumplattierung unter Verwendung einer elektrolytischen Lösung, die ein Sulfon und ein Magnesiumsalz aufweist, um eine Schicht aus aktivem Material bereitzustellen, die die Elektrode aufweist, wobei die Schicht aus aktivem Material mindestens Magnesium, Kohlenstoff, Sauerstoff, Schwefel und Halogen aufweist und eine Oberfläche aufweist, die einen einzelnen Peak bzw. Spitzenwert aufweist, der von Magnesium in einem Bereich von 40 eV bis 60 eV abgeleitet ist.

8. Verfahren zur Herstellung einer Elektrode nach Anspruch 7, wobei das Sulfon mindestens eines aufweist, das aus der Gruppe ausgewählt ist, die aus Ethylisopropylsulfon, Ethyl-n-Propylsulfon, Ethyl-Sek-Butylsulfon und Di-n-Propylsulfon besteht.

9. Verfahren zur Herstellung einer Elektrode nach Anspruch 7, wobei das Magnesiumsalz mindestens eines aufweist, das aus der Gruppe ausgewählt ist, die aus Magnesiumchlorid, Magnesiumbromid, Magnesiumiodid, Magnesiumperchlorat, Magnesiumtetrafluorborat, Magnesiumhexafluorophosphat, Magnesiumhexafluorarsenat, Magnesiumperfluoralkylsulfonat und Magnesiumperfluoralkylsulfonylimidat besteht.

## Revendications

1. Électrode comprenant une couche de matériau actif formée par dépôt électrochimique de magnésium au moyen d'une solution électrolytique comportant une sulfone et un sel de magnésium dissous dans la sulfone, ladite couche de matériau actif comprenant ainsi au moins du magnésium, du carbone, de l'oxygène, du soufre et un halogène, et ayant une surface présentant un seul pic dérivé du magnésium dans une gamme de 40 eV à 60 eV.

2. Électrode selon la revendication 1 qui présente, sur une région allant de la surface jusqu'à une profondeur de 200 nm, un seul pic dérivé du magnésium dans une gamme de 40 eV à 60 eV.

3. Électrode selon la revendication 1, dans laquelle un état oxydé du magnésium est sensiblement constant depuis la surface jusqu'à une profondeur de 200 nm.

4. Électrode selon la revendication 1, comprenant en outre un collecteur et la couche de matériau actif disposée sur le collecteur, la couche de matériau actif comportant au moins du magnésium, du carbone, de l'oxygène, du soufre et un halogène, et présentant, sur une profondeur allant d'une surface à l'autre de la couche de matériau actif, un seul pic dérivé du magnésium dans une gamme de 40 eV à 60 eV.

5. Électrode selon la revendication 1, comprenant en outre un collecteur et la couche de matériau actif disposée sur le collecteur, la couche de matériau actif comportant au moins du magnésium, du carbone, de l'oxygène, du soufre et un halogène, et un état oxydé du magnésium étant sensiblement constant d'une surface à l'autre de la couche de matériau actif.

6. Dispositif électrochimique comprenant une électrode positive (10), une électrode négative (20) et un électrolyte (30), l'électrode négative (20) comprenant une couche de matériau actif formée par dépôt électrochimique de magnésium au moyen d'une solution électrolytique comportant une sulfone et un sel de magnésium dissous dans la sulfone, ladite couche de matériau actif comprenant ainsi au moins du magnésium, du carbone, de l'oxygène, du soufre et un halogène, et ayant une surface présentant un seul pic dérivé du magnésium dans une gamme de 40 eV à 60 eV.

7. Procédé de production d'une électrode comprenant la réalisation d'un dépôt électrochimique de magnésium au moyen d'une solution électrolytique comportant une sulfone et un sel de magnésium pour obtenir une couche de matériau actif comprise par l'électrode, ladite couche de matériau actif comprenant au moins du magnésium, du carbone, de l'oxygène, du soufre et un halogène, et ayant une surface présentant un seul pic dérivé du magnésium dans une gamme de 40 eV à 60 eV.

8. Procédé de production d'une électrode selon la revendication 7, dans lequel la sulfone en comprend au moins une choisie dans le groupe constitué par l'éthylisopropylsulfone, l'éthyl-n-propylsulfone, l'éthyl-sec-butylsulfone, et la di-n-propylsulfone.

9. Procédé de production d'une électrode selon la revendication 7, dans lequel le sel de magnésium en comprend au moins un choisi dans le groupe constitué par le chlorure de magnésium, le bromure de magnésium, l'iodure de magnésium, le perchlorate de magnésium, le tétrafluoroborate de magnésium, l'hexafluorophosphate de magnésium, l'hexafluoroarsénate de magnésium, un perfluoroalkylsulfonate de magnésium, et un perfluoroalkylsulfonylimidate de magnésium.
